# NEUE EUROPÄISCHE PATENTSCHRIFT

(11) **EP 0 868 166 B2**
(45) Veröffentlichungstag und Bekanntmachung des Hinweises auf die Entscheidung über den Einspruch: **11.03.2009**
(45) Hinweis auf die Patenterteilung: 16.10.2002
(21) Anmeldenummer: 96943094.1
(22) Anmeldetag: 12.12.1996
(51) Int. Cl.: A61Q 5/08, A61K 8/22, A61K 8/39

(54) **BLONDIERMITTEL**
HAIR BLEACHING AGENTS
AGENTS D'OXYGENATION DES CHEVEUX

(30) Priorität: 21.12.1995 DE 19548132
(43) Veröffentlichungstag der Anmeldung: 07.10.1998
(73) Patentinhaber: Henkel AG & Co. KGaA, 40589 Düsseldorf (DE)
(72) Erfinder: HÖFFKES, Horst, D-40595 Düsseldorf (DE); NEUHAUS, Winifried, D-40822 Mettmann (DE)
(86) Internationale Anmeldenummer: PCT/EP1996/005555
(87) Internationale Veröffentlichungsnummer: WO 1997/023196

(56) Entgegenhaltungen:
- EP-A- 0 560 088
- EP-A- 0 663 205
- EP-A2- 0 574 696
- EP-A2- 0 650 719
- DE-A- 1 617 829
- DE-A- 3 814 356
- DE-A- 19 600 216
- DE-A- 19 600 705
- DE-U- 9 309 445
- DE-U- 9 315 532
- FR-A1- 2 715 065
- US- - 4 170 637
- US- - 5 279 313

## Beschreibung

Die Erfindung betrifft ein Blondiermittel für menschliche Haare, das in Pulverform vorliegt.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepaßt werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhellende Verfahren, die sogenannten Blondierverfahren, im wesentlichen nur dunkelblonde oder hellere Haare. Die Grundlagen der Blondierverfahren sind dem Fachmann bekannt und können in einschlägigen Monographien, z.B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg. Thieme Verlag, Stuttgart, New York, nachgelesen werden.

So werden üblicherweise feste oder pastenformige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die genannten Zubereitungen, die vor der Anwendung mit einer Wasserstoffperoxidlösung vermischt werden, werden im weiteren als "Blondiermittel" bzw. "Blondierpulver" bezeichnet. Alle aufgeführten Mengenangaben beziehen sich, soweit nicht anders ausgerührt, ausschließlich auf diese Zubereitungen.

Weder die pastenförmigen noch die pulverförmigen Blondiermittel, die heute auf dem Markt sind, können als optimal angesehen werden. Während die Blondierwirkung auf dem Haar als befriedigend bezeichnet werden kann, bestehen doch noch eine Reihe von Nachteilen und Problemen sowohl bei Herstellung als auch bei der Handhabung dieser Mittel. Bei pastenförmigen Mitteln, die aus Stabilitätsgründen hochviskos eingestellt werden, können insbesondere die Dosierung und das Mischungsverhalten in der Wasserstoffperoxidlösung noch nicht befriedigen. Bei pulverförmigen Mitteln stehen das Staubverhalten, sowohl bei der Konfektionierung als auch bei der Anwendung, sowie ebenfalls das Mischungsverhalten bei der Anwendung im Mittelpunkt der Verbesserungsbemühungen.

In der EP-B1-0 560 088 wurde beispielsweise vorgeschlagen, das Staubverhalten von Blondierpulvern durch Zugabe von Ölen oder flüssigen Wachsen zu verbessern. Diese Verbesserungen sollen durch Zugabe geringer Mengen, d.h. bis zu 2,5 Gew.-%, an nichtionischen Tensiden noch gesteigert werden.

Es wurde nun überraschenderweise gefunden, daß Blondierpulver mit vergleichbarem Staubverhalten aber signifikant besserem Mischungsverhalten erhalten werden, wenn diese Pulver größere Mengen an nichtionogenen oberflächenaktiven Substanzen gemäß Anspruch 1 enthalten.

Gegenstand der vorliegenden Erfindung sind daher Mittel zum Blondieren menschlicher Haare auf Basis mindestens einer festen Peroxoverbindung und mindestens eines festen Alkaliträgers, dadurch gekennzeichnet, daß sie als Pulver vorliegen und 4 - 20 Gew.-% einer nichtionogenen grenzflächenaktiven Substanz gemäß Anspruch 1 sowie bei Raumtemperatur feste Seifen enthalten.

Die erfindungsgemäßen Blondiermittel enthalten als erste zwingende Komponente eine feste Peroxoverbindung. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten.

Die Peroxoverbindungen sind in den erfindungsgemäßen Blondiermitteln bevorzugt in Mengen von 20-80 Gew.-%, insbesondere in Mengen von 40-70 Gew.-% enthalten.

Als weitere zwingende Komponente enthalten die erfindungsgemäßen Blondiermittel ein Alkalisierungsmittel, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall- und Erdalkalimetallhydroxyde, -carbonate, -hydrogencarbonate,
-hydroxycarbonate, -silikate, insbesondere -metasilikate, sowie Alkaliphosphate verwendet werden. In einer bevorzugten Ausführungsform enthalten die erfindungsgemäßen Blondierpulver mindestens zwei unterschiedliche Alkalisierungsmittel. Dabei können Mischungen beispielsweise aus einem Metasilikat und einem Hydroxycarbonat bevorzugt sein.

Die erfmdungsgemäßen Blondiermittel enthalten Alkalisierungsmittel bevorzugt in Mengen von 10-30 Gew.-%, insbesondere 15-25 Gew.-%.

Erfindungswesentlich ist die dritte zwingende Komponente der Blondierpulver, die nichtionogene oberflächenaktive Substanz gemäß Anspruch 1. Wenngleich die vorteilhaften Effekte bei einer Vielzahl von nichtionogenen oberflächenaktiven Substanzen auftreten, so haben sich doch solche als besonders geeignet erwiesen, die einen HLB-Wert von 5,0 und mehr besitzen.

Das Konzept des HLB-Wertes ist dem Fachmann bekannt. Es wird, auch in bezug auf die Definitionen, ausdrücklich auf die bekannten Monographien und Originalarbeiten verwiesen. Insbesondere auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten wird ausdrücklich Bezug genommen.

Erfindungsgemäß geeignete nichtionogene oberflächenaktive Substanzen sind unter den Vertretern der im folgenden beschriebenen Klassen, wobei vorzugsweise Substanzen mit einem HLB-Wert von mindestens 5,0 verwendet werden. Dabei sind Substanzen, die kommerziell als Feststoffe oder Flüssigkeiten in reiner Form erhältlich sind, wegen der einfacheren Verarbeitbarkeit besonders bevorzugt.

Eine erste Klasse stellen alkoxylierte Fettalkohole mit 8 bis 22, insbesondere 10.bis 16, Kohlenstoffatomen in der Fettalkylgruppe und 1 bis 30, insbesondere 1 bis 15, Ethylenoxid- und/oder Propylenoxid-Einheiten dar. Bevorzugte Fettalkylgruppen sind beispielsweise Lauryl-, Myristyl-, Cetyl-, aber auch Stearyl-, Isostearyl- und Oleylgruppen. Wenngleich die alkoxylierten Fettalkohole sowohl Ethylenoxid als auch Propylenoxid-Einheiten enthalten können, so kann es doch bevorzugt sein, Substanzen auszuwählen, die nur einen Typ dieser Gruppen, insbesondere nur Ethylenoxid-Einheiten, enthalten. Besonders bevorzugte Verbindungen dieser Klasse sind beispielsweise Laurylalkohol mit 2 bis 4 Ethylenoxid-Einheiten, Oleylund Cetylalkohol mit jeweils 5 bis 10 Ethylenoxideinheiten und Cetyl- und Stearylalkohol sowie deren Mischungen mit 10 bis 30 Ethylenoxideinheiten. Ein bevorzugter ethoxylierter und propoxylierter Fettalkohol ist das Handelsprodukt Aethoxal®B (Henkel), ein Laurylalkohol mit jeweils 5 Ethylenoxid- und Propylenoxideinheiten.

Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C1-C4-Alkyl-Ethers, "verschlossen". Ein Beispiel für eine solche Verbindung ist das Handelsprodukt Dehypon®O 054, ein Octanol + 4,5 Ethylenoxid-butylether.

Weiterhin können, wie in den bevorzugten Verbindungen bereits erwähnt, anstelle spezieller Fettalkohole auch Mischungen eingesetzt werden, wie sie beispielsweise aus natürlichen Fetten und Ölen gewonnen werden. Eine bevorzugte Fettalkoholmischung wird durch die übliche Aufarbeitung von Kokosölen erhalten.

Schließlich können Verbindungen, die Anlagerungsprodukte von Ethylenund/oder Propylenoxid an Fettalkohole darstellen, sowohl als Produkte mit einer "normalen" Homologenverteilung als auch in Form solcher Produkte mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder alkoholate als Katalysatoren verwendet werden. Die Verwendung von Produkten mit eingeengter Homologenveteilung kann bevorzugt sein.

Eine zweite Klasse nichtionogener grenzflächenaktiver Substanzen stellen alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten dar. Bevorzugte Fettsäuren sind beispielsweise Laurin-, Myristin-, Palmitin-, Stearin-, Isostearin- und Ölsäure. Wenngleich die alkoxylierten Fettsäuren sowohl Ethylenoxid als auch Propylenoxid-Einheiten enthalten können, so kann es doch bevorzugt sein, Substanzen auszuwählen, die nur einen Typ dieser Gruppen, insbesondere nur Ethylenoxid-Einheiten, enthalten. Besonders bevorzugte Verbindungen dieser Klasse sind beispielsweise Diethylenglykolmonostearat und -laurat sowie Tetraethylenglykolmonolaurat.

Eine dritte Klasse von geeigneten nichtionogenen grenzflächenaktiven Substanzen sind alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid.

Eine weitere Klasse von geeigneten nichtionogenen grenzflächenaktiven Substanzen sind Polyglycerinester und alkoxylierte Polyglycerinester. Bevorzugte Verbindungen dieser Klasse sind beispielsweise Poly (3)glycerindiisostearat (Handelsprodukt: Lameform®TGI (Henkel)) und Poly(2)glycerinpolyhydroxystearat (Handelsprodukt: Dehymuls®PGPH (Henkel)).

Eine weitere Klasse von geeigneten nichtionogenen grenzflächenaktiven Substanzen sind Sorbitan-Fettsäureester und alkoxylierte Sorbitan-Fettsäureester wie beispielsweise Sorbitanmonolaurat und Sorbitanmonolaurat + 20 Ethylenoxid (EO).

Eine weitere Klasse nichtionogener grenzflächenaktiver Verbindungen sind schließlich Alkylphenole und Alkylphenolalkoxylate mit 6 bis 21, insbesondere 6 bis 15, Kohlenstoffatomen in der Alkylkette und 0 bis 30 Ethylenoxidund/oder Propylenoxid-Einheiten. Bevorzugte Vertreter dieser Klasse sind beispielsweise Nonylphenol + 4 EO, Nonylphenol + 9 EO, Octylphenol + 3 EO und Octylphenol + 8 EO.

Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.

Als besonders vorteilhaft haben sich erfindungsgemäße Mittel erwiesen, die nichtionogene grenzflächenaktive Substanzen in Mengen von 5 bis 15 Gew.-% enthalten.

Weiterhin können die erfindungsgemäßen Blondiermittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.

Bevorzugte anionische Tenside sind Alkylsulfate, Ethercarbonsäuren mit 10 bis 18 C-Atomen in der Alkylgruppe und bis zu 12 Glykolethergruppen im Molekül.

Diese anionischen Tenside sollten bevorzugt in fester, insbesondere Pulverform vorliegen. Eine ganz besonders bevorzugte bei Raumtemperatur feste Seife ist Natriumstearat. Die bei Raumtemperatur festen Seifen liegen bevorzugt in Mengen von 5 bis 20 Gew.-%, insbesondere 10 bis 15 Gew.-.%, vor.

Als nichtionische Tenside eignen sich insbesondere C8-C22-Alkylmono- und oligoglycoside und deren ethoxylierte Analoga. Insbesondere die nichtethoxylierten Verbindungen, die zudem in Pulverform kommerziell erhältlich sind, haben sich als besonders geeignet erwiesen.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie das unter der Bezeichnung Dehyquart®F 75 in Abmischung mit Cetearylalkohle erhältliche Distearoylethylhydroxyethylammoniummethosulfat.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether und andere, als Feststoff stabile bzw. im Handel erhältliche Verbindungen,
- zwitterionische und amphotere Polymere, die als Feststoffe stabil bzw. bevorzugt als Handelsprodukte erhältlich sind,
- anionische Polymere wie beispielsweise Polyacrylsäuren, vernetzte Polyacrylsäuren und Vinylacetat/Crotonsäure-Copolymere, sofern diese als Feststoffe stabil bzw. bevorzugt im Handel erhältlich sind,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Farbstoffe zum Einfärben der Zubereitungen,
- Wirkstoffe wie Panthenol, Pantothensäure, Allantoin, Pyrrolidoncarbonsäuren und deren Salze,
- Cholesterin,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Carbonate, Hydrogencarbonate, Guanidine, Harnstoffe sowie primäre, sekundäre und tertiäre Phosphate,

Die Auswahl dieser weiteren Stoffe wird der Fachmann gemäß der gewünschten Eigenschaften der Mittel treffen.

Da sich gezeigt hat, daß auf die Anwesenheit insbesondere von größeren Mengen an Ölen und flüssigen Wachsen, wie beispielsweise Paraffinöl, verzichtet werden kann, können erfindungsgemäße Mittel, insbesondere wenn das Haar nicht übermäßig beschwert werden soll, in einer bevorzugten Ausführungsform ohne diese Öle und flüssigen Wachse konfektioniert werden. Dabei ist klarzustellen, daß der Begriff Öle die bekannten fetten und synthetischen Öle, nicht aber Parfümöle umfaßt, die selbstverständlich in geringen Mengen als Duftstoffe eingesetzt werden können.

Die Herstellung der erfindungsgemäßen Blondiermittel kann nach den üblichen, dem Fachmann bekannten Verfahren erfolgen.

Ein bevorzugtes Verfahren besteht darin, die anorganischen, als Feststoff vorliegenden Komponenten, gegebenenfalls nach Mischung z.B. in einem Drais-Mischer, vorzulegen und mit dem grenzflächenaktiven Mittel zu besprühen. Dies erfolgt bevorzugt bei Raumtemperatur, d. h. bei Temperaturen unterhalb von ca. 30 °C; lediglich wenn die gewählten staubbindenden Komponenten bei diesen Temperaturen nicht als Flüssigkeit vorliegen, wird man erhöhte Temperaturen anwenden.

Ein weiteres Herstellungsverfahren für die erfindungsgemäßen Blondiermittel ist das Mischen aller Komponenten und die anschließende Behandlung, bevorzugt bei erhöhten Temperaturen, im Wirbelbett

Schließlich ist es auch möglich, die erfindungsgemäßen Blondiermittel durch Vermahlen aller Komponenten in einer Kugelmühle, einer Ringwalzenmühle oder insbesondere einer Spindelmühle herzustellen.

Für die Anwendung der erfindungsgemäßen Mittel auf dem Haar werden die pulverfötmigen Blondiermittel unmittelbar vor dem Auftragen mit einer Wasserstoffperoxid-Lösung vermischt. Die Konzentration dieser Wasserstoflperoxid-Lösung wird einerseits von den gesetzlichen Vorgaben und andererseits von dem gewünschten Effekt bestimmt; in der Regel werden 6- bis 12prozentige Lösungen in Wasser verwendet. Die Mengenverhältnisse von Blondierpulver und Wasserstoffperoxid-Lösung liegen dabei üblicherweise im Bereich 1:1 bis 1:2, wobei ein Überschuß an Wasserstoffperoxid-Lösung insbesondere dann gewählt wird, wenn keine zu ausgeprägte Blondierwirkung erwünscht ist.

### Beispiele

Es wurden 5 Mischungen zubereitet, die jeweils die gleiche Menge an üblichen Blondiermittelkomponenten enthielten und sich nur durch die Komponente(n) zur Optimierung der Pulvereigenschaften unterschieden. Die Zusammensetzung dieser Mittel (in g) sind in Tabelle 1 angegeben:

| Komponenten | V1 | V2 | E1 | E2 | E3 |
|---|---|---|---|---|---|
| Ammoniumperoxidisulfat | 25 | 25 | 25 | 25 | 25 |
| Kaliumperoxidisulfat | 35 | 35 | 35 | 35 | 35 |
| Natriummetasilikat | 18 | 18 | 18 | 18 | 18 |
| Magnesiumhydroxycarbonat | 3 | 3 | 3 | 3 | 3 |
| Natriumstearat | 11 | 11 | 11 | 11 | 11 |
| pyrogenes Siliziumdioxid | 3,5 | 3,5 | 3,5 | 3,5 | 3,5 |
| Dinatriumdihydrogenethylendiamin- | | | | | |
| tetaacetat | 2 | 2 | 2 | 2 | 2 |
| Laurylalkohol + 2 EO | - | - | 10 | - | - |
| Laurylalkohol + 4 EO | 2,5 | 2,5 | - | 10 | - |
| Sorbitanmonolaurat | - | - | - | - | 10 |
| Paraffinöl perliq. | - | 10 | - | - | - |
| Gesamtmenge | 100 | 110 | 107,5 | 107,5 | 107,5 |
| Mischzeit [min] | 3 | 2 | 1 | 1 | 1 |

Die Rezepturen E1-E3 waren erfindungsgemäß, die Rezepturen V1 und V2 Vergleichsbeispiele.

Zur Untersuchung des Mischungsverhaltens wurden 50 g (V1) bzw. 55 g (V2, E1-E3) Pulver mit 50 ml einer 10%igen Wasserstoffperoxidlösung mit Hilfe eines Holzspatels in einer Mischwanne vermischt. Als Mischzeit wurde die Zeit bestimmt, nach der für das menschliche Auge eine homogene Mischung vorlag. Die Mischzeiten sind in Tabelle 1 ebenfalls aufgeführt (mit einem kommerziellen Blondierpulver in Granulatform waren zum Vergleich noch nach 10 Minuten grobkörnige Partikel zu erkennen).

Die an unterschiedlich stark pigmentierten Haaren durchgeführten Blondierungen zeigten für die Rezepturen V1, V2, E2 und E3 vergleichbare Ergebnisse; für Rezeptur E1 wurde eine bessere Blondierwirkung beobachtet.

## Patentansprüche

1. Mittel zum Blondieren menschlicher Haare auf Basis mindestens einer festen Peroxoverbindung und mindestens eines festen Alkaliträgers, **dadurch gekennzeichnet, daß** es als Pulver vorliegt und 4 - 20 Gew.-% einer nichtionogenen grenzflächenaktiven Substanz ausgewählt aus
- alkoxylierten Fettalkoholen und Fettsäuren mit 8 bis 22 Kohlenstoffatomen und 1 bis 30 Ethylenoxid- und/oder Propylenoxid-Einheiten, die eine Alkylendgruppe, insbesondere eine Methylgruppe, am Ende der Alkoxygruppenkette aufweisen können,
- alkoxylierten Mono-, Di- und Triglyceriden,
- Polyglycerinestern und alkoxylierten Polyglycerinestern,
- Sorbitan-Fettsäureestern und alkoxylierten Sorbitan-Festtsäureestern,
- Alkylphenolalkoxylaten mit 6 bis 21 Kohlenstoffatomen in der Alkylkette und 0 bis 30 Ethylenoxid- und/oder Propylenoxid-Einheiten,
sowie bei Raumtemperatur feste Seifen enthält.

2. Mittel nach Anspruch 1, **dadurch gekennzeichnet, daß** es eine anorganische feste Peroxoverbindung enthält.

3. Mittel nach Anspruch 1 oder 2, **dadurch gekennzeichnet, daß** die nichtionogene grenzflächenaktive Substanz einen HLB-Wert von mindestens 5,0 aufweist.

4. Mittel nach einem der Ansprüche 1 bis 3, **dadurch gekennzeichnet, daß** das nichtionogene Tensid ausgewählt ist aus
- alkoxylierten Fettalkoholen und Fettsäuren mit 8 bis 22, insbesondere 10 bis 16 Kohlenstoffatomen und 1 bis 15 Ethylenoxid- und/oder Propylenoxid-Einheiten und
- Alkylphenolalkoxylaten mit 6 bis 15 Kohlenstoffatomen in der Alkylkette und 1 bis 15 Ethylenoxid- und/oder Propylenoxid-Einheiten.

5. Mittel nach einem der Ansprüche 1 bis 4, **dadurch gekennzeichnet, daß** es 5 - 15 Gew.-% nichtionogene grenzflächenaktive Substanzen enthält.

6. Mittel nach einem der Ansprüche 1 bis 5, **dadurch gekennzeichnet, daß** es die bei Raumtemperatur feste Seifen, insbesondere Natriumstearat, in Mengen von 5 - 20 Gew.-%, insbesondere 10-15 Gew.-%, bezogen auf das gesamte Pulver, enthält.

7. Mittel nach einem der Ansprüche 1 bis 6, **dadurch gekennzeichnet, daß** es frei von Ölen und flüssigen Wachsen ist, wobei Parfümöle ausgenommen sind.

## Claims

1. A preparation for blonding human hair based on at least one solid peroxo compound and at least one solid alkali carrier, **characterized in that** it is formulated as a powder and contains
4 to 20% by weight of a nonionic surfactant selected from
- alkoxylated fatty alcohols and fatty acids containing 8 to 22 carbon atoms and 1 to 30 ethylene oxide and/or propylene oxide units which may have a terminal alkyl group, more particularly a methyl group, at the end of the alkoxy group chain,
- alkoxylated mono-, di- and triglycerides,
- polyglycerol esters and alkoxylated polyglycerol esters,
- sorbitan fatty acid esters and alkoxylated sorbitan fatty acid esters,
- alkylphenol alkoxylates containing 6 to 21 carbon atoms in the alkyl chain and 0 to 30 ethylene oxide and/or propylene oxide units and
soaps solid at room temperature.

2. A preparation as claimed in claim 1, **characterized in that** it contains an inorganic solid peroxo compound.

3. A preparation as claimed in claim 1 or 2, **characterized in that** the nonionic surfactant has an HLB value of at least 5.0.

4. A preparation as claimed in claim 1 to 3, **characterized in that** the nonionic surfactant is selected from
- alkoxylated fatty alcohols and fatty acids containing 8 to 22 and more particularly 10 to 16 carbon atoms and 1 to 15 ethylene oxide and/or propylene oxide units and
- alkylphenol alkoxylates containing 6 to 15 carbon atoms in the alkyl chain and 1 to 15 ethylene oxide and/or propylene oxide units.

5. A preparation as claimed in any of claims 1 to 4, **characterized in that** it contains 5 to 15% by weight of nonionic surfactants.

6. A preparation as claimed in any of claims 1 to 5, **characterized in that** it contains the soaps solid at room temperature, more particularly sodium stearate, in quantities of 5 to 20% by weight and more particularly in quantities of 10 to 15% by weight, based on the powder as a whole.

7. A preparation as claimed in any of claims 1 to 6, **characterized in that** it is free from oils and liquid waxes except perfume oils.

## Revendications

1. Agent de pour blondir les cheveux humains à base d'au moins un composé peroxo solide et d'au moins un vecteur d'alcali solide,
**caractérisé en ce qu'**
il se présente sous forme de poudre et qu'il contient de 4 à 20 % en poids d'une substance active sur la tension superficielle non-ionogène choisie parmi:
- les alcools gras alkoxylés et les acides gras ayant de 8 à 22 atomes de carbone et de 1 à 30 éléments d'oxyde d'éthylène et/ou d'oxyde de propylène, qui peuvent posséder un groupe terminal alkyle, en particulier un groupe méthyle, à l'extrémité de la chaîne des groupes alkoxy,
- des mono-, di- et triglycérides alkoxylés,
- des esters de polyglycérol et des esters des polyglycérol alkoxylés,
- des esters d'acide gras de sorbitanne et des esters d'acide gras des sorbitanne alkoxylés,
- des alkoxylates d'alkylphénols ayant de 6 à 21 atomes de carbone dans la chaîne alkyle et de 0 a 30 éléments d'oxyde d'éthylène et/ou d'oxyde de propylène
et des savons solides à temperature ambiante.

2. Agent selon la revendication 1,
**caractérisé en ce qu'**
il contient un composé peroxo inorganique, solide.

3. Agent selon l'une quelconque des revendications 1 ou 2,
**caractérisé en ce que** la substance active sur la tension superficielle non ionogène possède une valcur d'HLB d'au moins 5,0.

4. Agent selon l'une quelconque de revendications 1 à 3,
**caratérisé en ce que**
l'agent tensioactif non ionogène est choisi parmi,
- les alcools gras et les acides gras alkoxylés ayant de 8 à 22, en particulier de 10 à 16 atomes de carbone et de 1 à 15 éléments d'oxyde d'éthylène et/ou d'oxyde de propylène,
- des alkoxylates d'alkylphénol ayant de 6 à 15 atomes de carbone dans la chaîne alkyle et de 1 à 15 éléments d'oxyde d'éthylène et/ou d'oxyde de propyléne.

5. Agent selon l'une quelconque des revendications 1 à 4,
**caractérisé en ce qu'**
il contient de 5 à 15 % en poids de substances actives sur la tension superficielle non ionogène.

6. Agent selon l'une quelconque des revendications 1 à 5,
**caractérisé en ce qu'**
il contient les savons solides à température ambiante, en particulier du stéarate de sodium en quantités allant de 5 à 20 % en poids, en particulier de 10 à 15 % en poids rapporté à la totalité de la poudre.

7. Agent selon l'une quelconque des revendications 1 à 6,
**caractérisé en ce qu'**
il est dépourvu d'huiles et de cires liquides, à l'exception d'essences de parfum.
